Europäisches Patentamt

European Patent Office

Office européen des brevets

(19)

(11) Veröffentlichungsnummer: **0 001 087**
**B1**

(12) **EUROPÄISCHE PATENTSCHRIFT**

(45) Veröffentlichungstag der Patentschrift:
07.01.81

(21) Anmeldenummer: **78100776.0**

(22) Anmeldetag: **29.08.78**

(51) Int. Cl.³: **C 07 C 143/665**, C 07 C 97/24,
C 07 C 139/00

(54) Verfahren zur Herstellung von 1-Amino-4-bromanthrachinon-2-sulfonsäure.

(30) Priorität: **10.09.77 DE 2740889**

(43) Veröffentlichungstag der Anmeldung:
**21.03.79 Patentblatt 79/6**

(45) Bekanntmachung des Hinweises auf die Patenterteilung:
**07.01.81 Patentblatt 81/1**

(84) Benannte Vertragsstaaten:
**BE CH DE FR GB LU NL**

(56) Entgegenhaltungen:
**DE-B-1 135 495**
**FR-A-653 159**
**GB-A-664 839**
**US-A-2 590 247**
**CHEMICAL ABSTRACTS, Vol. 60, Czech. 105, 953,
15. Dezember 1962, LADISLAV HRUSKA, THEODOR
PFLIEGEL, HANA TAEUBLOVA
CHEMICAL ABSTRACTS, Vol. 65, Czech. 115, 650,
15. August 1965**

(73) Patentinhaber: **BAYER AG, Zentralbereich Patente,
Marken und Lizenzen, D-5090 Leverkusen 1, Bayerwerk
(DE)**

(72) Erfinder: **Berg, Gerhard, Dr., Tempelhofer Strasse 46,
D-5090 Leverkusen 1 (DE)**
Erfinder: **Hohmann, Walter, Dr., Fontanestrasse 17,
D-5090 Leverkusen 1 (DE)**
Erfinder: **Reubke, Karl-Julius, Dr., Rybnikerstrasse 10,
D-5000 Köln 80 (DE)**
Erfinder: **Wunderlich, Klaus, Dr., Carl-Rumpff-Strasse 21,
D-5090 Leverkusen (DE)**

## Verfahren zur Herstellung von 1-Amino-4-bromanthrachinon-2-sulfonsäure

Die vorliegende Erfindung betrifft ein Verfahren zur Herstellung von 1-Amino-4-bromanthrachinon-2-sulfonsäure oder deren Alkalisalze aus verunreinigtem 1-Nitroanthrachinon.

Die Herstellung von 1-Amino-4-bromanthrachinon-2-sulfonsäure (im folgenden als Bromaminsäure bezeichnet) wird üblicherweise in einem 2-Stufen-Verfahren durchgeführt, wobei in Stufe 1 die Sulfonierung des 1-Amino-anthrachinons mit Chlorsulfonsäure in einem mit Wasser nicht mischbaren inerten organischen Lösungsmittel erfolgt. Nach Abtrennen vom organischen Lösungsmittel wird dann in neutraler wässriger Lösung (vgl. z.B. US-PS 3 428 659) oder in schwefelsaurer Lösung (vgl. z.B. japanische Offenlegungsschrift 49 076 848) bromiert.

Ein anderer zweistufiger Weg besteht darin, 1-Amino-anthrachinon zunächst in $SO_3$-haltiger Schwefelsäure zu sulfonieren (vgl. DT-PS 2 63 395 und 4 84 997), die erhaltene 1-Amino-anthrachinon-2-sulfonsäure zu isolieren und in wässrigem Medium zu bromieren (vgl. z.B. FIAT 1 313 II, S. 214, US-PS 2 413 790, 2 440 760 und 2 503 254, DT-AS 2 303 246).

Gemäss dem in der DT-OS 2 551 767 beschriebenen Verfahren wird während der Sulfonierung noch Borsäure zugesetzt; die Bromierung erfolgt in stark verdünnter wässriger Schwefelsäure.

Bei den genannten Verfahren muss die Aminosulfonsäure zwischenisoliert oder die Sulfonierungsschmelze vor der Bromierung stark mit Wasser verdünnt werden.

Die Sulfierung des 1-Aminoanthrachinons mit rauchender Schwefelsäure, gegebenenfalls in Gegenwart von $SO_3$, ist auch in der DE-AS 11 35 495, der GB-PS 664 839, der französischen Patentschrift 653 159 und in Chemical Abstracts, Vol. 60, 2874b beschrieben. Die dort beschriebenen Sulfonierungsreaktionen gehen alle vom 1-Aminoanthrachinon aus und werden z.T. in Gegenwart von säurebindenden Zusätzen, wie Borsäure und tertiären Stickstoffbasen, durchgeführt.

Die Bromierung der 1-Aminoanthrachinon-2-sulfonsäure ist noch beschrieben in Chemical Abstract, Vol. 65, 670e, der US-PS 2 590 247 und in der GB-PS 664 839. Man geht dabei entweder von der 1-Aminoanthrachinon-2-sulfonsäure oder, wie in der GB-PS 664 839 beschrieben, von 1-Aminoanthrachinon aus, das zunächst sulfoniert und dann bromiert wird. Die Bromierungsreaktionen werden unterschiedlich durchgeführt. Während gemäss der US-PS 2 590 247 eine wässrige Lösung von 1-Aminoanthrachinon-2-sulfonsäure mit Bromdampf und Luft in Gegenwart anorganischer Salze bromiert wird, führt man die Bromierung nach Chemical Abstracts mit flüssigem Brom in Gegenwart einer cyclischen Stickstoffbase oder, wie in der GB-PS beschrieben, mit einem, die 1-Aminoanthrachinon-2-sulfonsäure enthaltenen Gemisch, das noch eine tertiäre Stickstoffbase enthält, durch.

Um bei diesen Verfahren die Bromaminsäure in einer für die Farbstoffproduktion brauchbaren Qualität erhalten zu können, ist es erforderlich, von möglichst reinem 1-Aminoanthrachinon auszugehen (vgl. z.B. DE-OS 2 206 960 und Org. Poluprod. i. Krasiteli, Moskau, 1969, Nr. 4, Seiten 70–79). Ausserdem gestaltet sich die Isolierung der Bromaminsäure technisch aufwendig, was die Wirtschaftlichkeit der Verfahren beeinträchtigt.

Es wurde überraschenderweise gefunden, dass man 1-Amino-4-bromanthrachinon-2-sulfonsäure oder deren Alkalisalze in hoher Ausbeute und guter Qualität erhält, wenn man

a) verunreinigtes 1-Nitroanthrachinon mit einem Reingehalt von 70–90% an 1-Nitroanthrachinon durch Reduktion mit Natriumsulfid und/oder Natriumhydrogensulfid oder durch Austausch der Nitrogruppe mit Ammoniak unter Druck in 1-Amino-anthrachinon überführt, anschliessend

b) das rohe 1-Amino-anthrachinon bei Temperaturen im Bereich von 100 bis 150°C zuerst mit Oleum unter Zusatz von Alkalimetallsulfaten und dann bei Temperaturen im Bereich von 60 bis 100°C mit Brom behandelt und danach

c) die 1-Amino-4-bromanthrachinon-2-sulfonsäure oder deren Alkalisalze aus dem Reaktionsgemisch durch Einstellen der Schwefelsäure-Konzentration auf einen Schwefelsäure-Gehalt von 60 bis 85 Gew.-% oder durch Einrühren des Reaktionsgemisches in gegebenenfalls alkalisulfathaltiges Wasser ausfällt, abfiltriert und gegebenenfalls die 1-Amino-4-bromanthrachinon-2-sulfonsäure oder deren Alkalisalze aus Wasser umlöst.

Es ist eine Besonderheit des erfindungsgemässen Verfahrens, verunreinigtes 1-Nitroanthrachinon einzusetzen, das einen Reingehalt von 70–90% an 1-Nitroanthrachinon besitzt.

Das einzusetzende rohe 1-Nitroanthrachinon kann an sich nach beliebigen Verfahren erhalten werden, sofern es die obengenannten Reinheitsanforderungen erfüllt. Beispielsweise kann es nach den in der DE-AS 2 039 822 beschriebenen Verfahren erhalten werden. Bevorzugt setzt man jedoch ein 1-Nitroanthrachinon ein, das nach den Nitrierungsvorschriften der Beispiele 1c, 2c, 3c oder nach den in der DE-OS 2 233 185 beschriebenen Verfahren erhalten wird. Das so hergestellte 1-Nitroanthrachinon enthält etwa 70 bis 80%, vorzugsweise 72 bis 77%, 1-Nitroanthrachinon, ca. 5 bis 10%, vorzugsweise 6 bis 8%, $\alpha,\alpha$-Dinitroanthrachinone, etwa jeweils ca. 4 bis 10%, vorzugsweise 5 bis 8%, $\alpha,\beta$-Dinitroanthrachinone und 2-Nitroanthrachinon sowie weniger als 5%, vorzugsweise weniger als 3%, Anthrachinon.

Die Nitroanthrachinon-Gemische können in Verfahrensschritt a) nach an sich bekannten Verfahren, z.B. durch Behandlung mit Reduktionsmitteln oder durch Substitution der Nitrogruppe durch Ammoniak, in entsprechende Amin-Gemische überführt werden.

Die Reduktion der Nitrogruppe kann z.B. mit katalytisch aktiviertem Wasserstoff, mit Hydrazinhydrat, vorzugsweise aber mit Natriumsulfid und/oder Natriumhydrogensulfid erfolgen (vgl. z.B. DT-OSen 2 340 114, 2 425 814, 2 348 102, 2 164 304, US-PS 2 874 168).

Der Austausch der Nitrogruppe durch die Aminogruppe kann durch Einwirkung von Ammoniak oder den Salzen des Ammoniaks in Wasser und/ oder in Gegenwart inerter organischer Lösungsmittel, gegebenenfalls unter Zusatz von Säure abfangenden Substanzen, wie Soda oder Alkalibicarbonaten bei erhöhter Temperatur, vorzugsweise unter Druck, erfolgen (vgl. z.B. DT-OSen 2 211 411, 2 409 542, 2 526 657).

Führt man den Nitrogruppen-Austausch in Wasser durch, so bekommt man ein Amingemisch, dessen Zusammensetzung unter Berücksichtigung der Stöchiometrie im wesentlichen der Zusammensetzung des Nitroanthrachinon-Gemisches entspricht.

Man kann aber auch die Reduktion so durchführen, dass ein partiell gereinigtes 1-Amino-anthrachinon entsteht, wie dies in den Beispielen 3 und 5 für eine Lösungsmittelbehandlung auf der Aminstufe oder eine Sufitbehandlung auf der Nitrostufe beschrieben ist. Solche Vorreinigungen können praktisch im Zuge der notwendigen Reaktionsschritte des beanspruchten Verfahrens ohne nennenswerten zusätzlichen Aufwand eingebaut werden. Jedoch ist eine derartige Vorreinigung nicht erfindungswesentlich, da auch gute Ausbeuten und Qualitäten an Bromaminsäure ohne derartige Operation erhalten werden können.

Andererseits kann ein durch eine derartige Behandlung resultierender erhöhter Gehalt an Nitro- oder Aminoanthrachinon im jeweiligen Gemisch die spätere Isolierung der Bromaminsäure erleichtern. Einen hohen Gehalt an 1-Nitroanthrachinon im einzusetzenden 1-Nitroanthrachinon-Gemisch kann man beispielsweise leicht durch Vorreinigen der bei einer Mischsäure-Nitrierung erhaltenen Nitroanthrachinon-Gemische, z.B. mit Lösungsmitteln wie in Beispiel 4 beschrieben, erhalten.

Gemäss Verfahrensschritt b) wird das nach a) erhaltene 1-Amino-anthrachinon-Gemisch in etwa 5- bis 40%igem Oleum, vorzugsweise in 10- bis 25%igem Oleum, bei Temperaturen im Bereich von 100 bis 150°C, vorzugsweise bei 120 bis 140°C, behandelt. Da bei dieser Temperatur das Oleum auf Aminoanthrachinone oxidierend wirkt, beispielsweise unter Hydroxylierung der 4-Position, setzt man zur Vermeidung dieser Reaktion wasserfreie Alkalimetallsulfate zu. Der Alkalimetallsulfat-Zusatz erschwert zudem den Eintritt einer zweiten Sulfonsäuregruppe. Als Alkalimetallsulfate verwendet man üblicherweise wasserfreies Natriumsulfat und/oder Kaliumsulfat. Man setzt etwa 0,4 bis 1,4 Teile, vorzugsweise 0,6 bis 1,2 Teile, Alkalimetallsulfat pro Teil Aminoanthrachinone dem Reaktionsgemisch zu.

An Oleum gibt man etwa die 2- bis 5fache, bevorzugt die 3- bis 4fache, Gewichtsmenge Oleum, bezogen auf Aminoanthrachinone, dem nach Schritt a) erhaltenen Gemisch zu. Die Oleum-Menge, der $SO_3$-Gehalt des Oleum und die Reaktionstemperatur stehen dabei in einem bestimmten Zusammenhang. Es werden bevorzugt niedrige $SO_3$-Gehalte mit hohen Oleum-Mengen und mittleren bis hohen Temperaturen, sowie hohe $SO_3$-Gehalte mit niedrigen Oleum-Mengen und niedrigen bis mittleren Temperaturen kombiniert.

Für die Sulfonierung benötigt man im allgemeinen etwa 1 bis 10 Stunden, vorzugsweise 2 bis 6 Stunden. Das nach Schritt a) erhaltene Gemisch wird so praktisch vollständig in ein Gemisch aus überwiegend 1-Amino-anthrachinon-2-sulfonsäure und 1-Amino-anthrachinon-2,4-disulfonsäure übergeführt. Letzteres bildet sich vornehmlich in der Endphase der Sulfierung, in der die Bildung der 1-Aminoanthrachinon-2-sulfonsäure nur noch langsam fortschreitet. Die Bildung der 2,4-Disulfonsäure ist für den weiteren Reaktionsablauf nicht kritisch, weil sie bei der anschliessenden Bromierung ganz überwiegend in Bromaminsäure übergeführt wird. Da aber auch in geringem Umfang 1-Amino-2,4-dibrom-anthrachinon und 1-Amino-2-brom-anthrachinon-4-sulfonsäure entstehen, ist es ratsam, die Sulfierung nicht bis zum völligen Verschwinden des Aminoanthrachinons durchzuführen, sondern dann abzubrechen, wenn noch etwa 1 bis 5%, vorzugsweise 2 bis 3%, Aminoanthrachinone im Gemisch nachweisbar sind. Wie sich chromatographisch nachweisen lässt, werden bei der Sulfierung die vorhandenen Diaminoanthrachinone sowie das 2-Amino-anthrachinon in verschiedene bromierte Mono- und Disulfonsäure-Derivate übergeführt.

Die Bromierung der Anthrachinonsulfonsäuren erfolgt ohne vorheriges Verdünnen mit Wasser direkt nach der Sulfierung im Gemisch durch Einwirkung von Brom bei Temperaturen im Bereich von etwa 60 bis 100°C, vorzugsweise bei 70 bis 90°C. Vor der Bromzugabe muss das Reaktionsgemisch auf diese Temperaturen abgekühlt werden.

Vorteilhaft für die Bromierung ist der Zusatz eines üblichen Halogenierungskatalysators wie Jod. Der Halogenierungskatalysator kann in Mengen von etwa 0,01 bis 3 Gew.-%, bevorzugt von etwa 0,1 bis 1 Gew.-%, bezogen auf eingesetzte 1-Amino-anthrachinone, zugegeben werden.

Da ein grosser Teil des bei der Bromierung entstehenden Bromwasserstoffs im Reaktionsgemisch zu Brom rückoxidiert wird, wird weniger als 1 Moläquivalent Brom pro Mol eingesetztem 1-Aminoanthrachinon verbraucht. Man benötigt mindestens 0,5 Moläquivalente, im allgemeinen etwa 0,6 bis 0,9 Moläquivalente, Brom, bezogen auf 1 Mol 1-Amino-Anthrachinon.

Die Bromierung kann drucklos unter Rückflusskühlung oder unter Druck durchgeführt werden. Vorteilhafterweise arbeitet man drucklos, gelegentlich kann es jedoch günstig sein, unter Druck bis etwa 3 bar zu arbeiten.

Der Verlauf der Bromierung lässt sich chromatographisch gut verfolgen. Im bevorzugten Temperaturbereich ist die Bromierung nach etwa 4 bis 16 Stunden beendet. Vor der Aufarbeitung ist es zweckmässig, überschüssiges Brom im Vakuum abzuziehen, um einerseits Geruchsbelästigungen, andererseits Nebenreaktionen durch Bromeinwirkung in Gegenwart von Wasser zu vermeiden.

Die Isolierung der Bromaminsäure oder deren Alkalisalze gemäss Verfahrensschritt c) kann auf verschiedene Weise erfolgen.

Einmal kann das Reaktionsgemisch – gegebenenfalls nach vorheriger Zugabe von Schwefelsäure verschiedener Konzentrationen (etwa 30–70 Gew.-%ige Schwefelsäure) oder nach vorsichtigem Zutropfen von Wasser – unter Rühren in vorgelegtes Wasser oder Eiswasser gegeben werden. Aus der sauren Suspension isoliert man dann die noch Nebenprodukte enthaltende Bromaminsäure – gegebenenfalls nach Zusatz von Alkalimetallsalzen – durch Absaugen. Die so erhaltene rohe Bromaminsäure enthält neben etwa 2 bis 5% unsulfonierten Amino-anthrachinonen, wie 1-Amino-2- bzw. -4-brom-anthrachinon und 1-Amino-2-4-dibrom-anthrachinon, etwa 1% 1-Amino-anthrachinon-2-sulfonsäure und eine grössere Menge (etwa 3–10%) einer Mischung bromierter anderer Aminoanthrachinon-sulfonsäure-Derivate.

Zur Abtrennung der wasserunlöslichen Aminoanthrachinone hat sich eine Klärung in saurem Medium, beispielsweise bei pH 3, oder eine alkalische Klärung, beispielsweise bei pH 9, bewährt. Die Klärung geschieht vorteilhafterweise in Gegenwart von Aktivkohle und/oder Kieselgel. Aus dem Filtrat der Klärung kann die Bromaminsäure durch Aussalzen, beispielsweise mit Natrium- und/oder Kaliumchlorid bzw. Natrium- und/oder Kaliumsulfat, in Form ihres Alkalisalzes ausgefällt werden. Man isoliert das Alkalisalz z.B. durch Absaugen in einer Nutsche, wäscht den Nutschkuchen mit verdünnter Salzlösung (ca. 1 bis 5%ig) und trocknet den Nutschkuchen. In dem Filtrat verbleibt der ganz überwiegende Anteil der bromierten Sulfierungsprodukte, die aus den Verunreinigungen des rohen 1-Amino-anthrachinons stammen. Das erhaltene Alkalisalz der Bromaminsäure ist von hoher Reinheit (>90%ig). Als Verunreinigung enthält das Salz der Bromaminsäure lediglich etwa 1% unsulfonierte Aminoanthrachinone, etwa 1% 1-Amino-anthrachinon-2-sulfonsäure und etwa 0,5 bis 2% andere (bromierte) Aminoanthrachinon-sulfonsäure-Derivate. Der an 100% fehlende Anteil besteht aus Alkalimetallsalzen und/oder Wasser. Der Bromaminsäureanteil bezogen auf organische Anteile liegt über 95%.

Eine weitere Aufarbeitungsmethode für die Bromaminsäure kann wie folgt durchgeführt werden.

Man verdünnt die Bromaminsäure-Schmelze durch Zugabe von Wasser oder verdünnter wässriger Schwefelsäure so, dass eine etwa 60 bis 85 Gew.-%ige, vorzugsweise 65 bis 80 Gew.-%ige, Schwefelsäure entsteht, filtriert das ausgefallene Sulfat der Bromaminsäure ab und verdrängt die Mutterlauge aus dem Nutschkuchen durch Wäsche mit verdünnter wässriger Schwefelsäure (Gehalt an $H_2SO_4$ >30%, vorzugsweise >50%). Das mit Schwefelsäure gewaschene Sulfat der Bromaminsäure wird durch Wäsche mit Wasser und/oder sulfathaltigem Wasser hydrolysiert, die Schwefelsäure entfernt und die Bromaminsäure anschliessend getrocknet.

Eine besonders gut filtrierbare und reine Form der Bromaminsäure erhält man, wenn man so verdünnt, dass die Bromaminsäureschmelze in vorgelegte verdünnte Schwefelsäure (vorzugsweise mit 20–40% $H_2SO_4$-Gehalt) eindosiert wird. Das Verdünnen mit Wasser oder wasserhaltiger Schwefelsäure erfolgt bei erhöhter Temperatur, vorzugsweise bei etwa 70 bis 100 °C, die Filtration bei etwa 30 bis 70 °C.

Die Verdünnung kann innerhalb etwa ½ bis 3 Stunden, vorzugsweise innerhalb 1 bis 2 Stunden, vorgenommen werden. Es ist zweckmässig, vor der Filtration bei der Filtrationstemperatur etwa eine ½ bis 10 Stunden nachzurühren.

Die Filtrationstemperatur und die Nachrührzeiten hängen dabei von der Art und Menge der Nebenprodukte ab.

Als Regel kann gelten, je mehr Verunreinigungen vorhanden sind, desto mehr muss die Filtrationstemperatur innerhalb des angegebenen Bereiches erhöht werden. Mit zunehmender Verunreinigung – die Einstellung des Kristallisationsgleichgewichts für die Bromaminsäure erfolgt langsamer – sind häufig längere Nachrührzeiten innerhalb des angegebenen Zeitraums erforderlich.

Man erhält im allgemeinen eine Bromaminsäure-Qualität, die derjenigen der zuvor beschriebenen Aufarbeitungsvariante entspricht. Bei schlecht filtrierender oder auswaschbarer Bromaminsäure kann es vorkommen, dass die Qualität erst erreicht wird, wenn an die Filtration und Wäsche eine Umlösung nach der zuvor beschriebenen Art angeschlossen wird.

Nach dem erfindungsgemässen Verfahren erhält man die Bromaminsäure in einer Ausbeute von etwa 65 bis etwa 80% der Theorie, bezogen auf 1-Nitro-anthrachinon. Die Bromaminsäure wird dabei in einer Reinheit von etwa 80 bis 90, bevorzugt 83 bis 86%, erhalten, bezogen auf Molgewicht: 382. Ihr Anteil an der organischen Substanz beträgt über 95%.

Das erfindungsgemässe Verfahren zeichnet sich im Vergleich zu bekannten Verfahren durch folgende Vorteile aus:

Das erfindungsgemässe Verfahren lässt sich ausgehend von verunreinigtem 1-Nitro-anthrachinon durchführen. Dabei kann 1-Nitro-anthrachinon ohne weitere Reinigung so eingesetzt werden, wie es bei einer Mono-Nitrierung durch Ausfällung aller Anthrachinon-Anteile erhalten wird.

Weiterhin kann 1-Amino-anthrachinon ohne weitere Reinigung so eingesetzt werden, wie es bei einer Reduktion oder dem Nitro-Amino-Gruppen-Austausch des rohen 1-Nitro-anthrachinons erhalten wird.

Daneben ist es von ausserordentlichem Vorteil, dass aus dem rohen 1-Amino-anthrachinon-Gemisch sich ohne weitere Isolierung der Zwischenprodukte die Bromaminsäure durch Sulfierung und Bromierung herstellen lässt (Eintopfverfahren). Dadurch gestaltet sich das erfindungsgemässe Verfahren besonders wirtschaftlich.

Ausserdem lässt sich die Isolierung der Bromaminsäure umweltfreundlich und ökonomisch durchführen, da die Isolierung der Bromaminsäure aus konzentrierter (70 bis 90 Gew.-%ige) Schwefelsäure erfolgen kann. Dadurch ist es möglich, entweder aus den Mutterlaugen die Schwefelsäure durch geeignete Massnahmen, z.B. in einer Plinke-Anlage zu regenerieren, ohne dass grosse Mengen Wasser abdestilliert werden müssen oder die Mutterlaugen ganz oder teilweise mitsamt den gelösten Verunreinigungen nach vorheriger Aufkonzentrierung mittels Oleum als Reaktionsschwefelsäure wieder einzusetzen. Abwasserprobleme entstehen dann nicht mehr oder nur in untergeordnetem Masse.

Bromaminsäure ist ein wichtiges Zwischenprodukt für zahlreiche wertvolle Anthrachinonfarbstoffe, vgl. Colour Index 62 125, 62 130, 62 070.

Die folgenden Beispiele sollen die Erfindung näher erläutern, dabei bedeuten Teile Gewichtsteile, sofern nicht ausdrücklich anderes vermerkt ist. Die Gehaltsangaben beruhen auf einer Bestimmung organisch gebundenen Broms in Kombination mit quantitativer Säulen- und Dünnschichtchromatographie.

Beispiel 1

a) In eine Mischung, bestehend aus 80 ml Oleum (20%ig) und 40 g wasserfreiem $Na_2SO_4$, werden innerhalb ½ Stunde 45 g 1-Amino-anthrachinon (Gehalt: 72,5%) eingetragen. Dabei steigt die Temperatur auf 130 °C an. Die Mischung wird bei dieser Temperatur so lange gehalten, bis in einer entnommenen Probe weniger als 3% 1-Amino-anthrachinon nachweisbar sind (ca. 4–5 Stunden erforderlich). Nun kühlt man auf Raumtemperatur ab, gibt 1 g fein gepulvertes Jod zu, tropft bei Raumtemperatur innerhalb 30 Minuten 8 ml Brom zu, erwärmt innerhalb 30 Minuten auf 80 °C und hält bei dieser Temperatur so lange, bis in einer entnommenen Probe nur noch spurenweise 1-Amino-anthrachinon-2-sulfonsäure nachweisbar ist (ca. 7–10 Stunden erforderlich).

Nun wird überschüssiges Brom im Vakuum abgezogen, das verbleibende Reaktionsgemisch in 2 l Wasser eingerührt. Zur Lösung gibt man portionsweise 320 g $Na_2SO_4$ zu und verrührt während 2 Stunden bei Raumtemperatur. Man saugt ab und wäscht mit 10%iger $Na_2SO_4$-Lösung nahezu neutral. Zur Abtrennung der unsulfonierten Aminoanthrachinonanteile wird das Nutschgut in 800 ml Wasser bei 95 °C verrührt, 4 g Aktivkohle zugesetzt, durch Zugabe von Soda auf pH 9–10 gestellt und bei 90 °C filtriert. Aus dem Klärfiltrat wird das Na-Salz der Bromaminsäure mit 20 g $Na_2SO_4$ ausgesalzen, über Nacht kalt gerührt, abfiltriert, mit 1%iger $Na_2SO_4$-Lösung gewaschen und getrocknet.

Erhalten werden 45 g Bromaminsäure folgender Analyse:[1]

1) Die Analysenzahlen beziehen sich hier und in der Folge auf die freien Sulfonsäuren (auch wenn deren Alkalimetallsalze vorliegen)

| | |
|---|---|
| 84,1% | 1-Amino-4-brom-anthrachinon-2-sulfonsäure |
| 1,4% | unsulfonierte Aminoanthrachinone |
| 1,0% | 1-Amino-anthrachinon-2-sulfonsäure |
| 0,9% | andere Anthrachinonsulfonsäuren |
| 7,0% | Wasser. |

Die Ausbeute beträgt demnach 72% der Theorie.

b) Das in a) eingesetzte 1-Amino-anthrachinon wurde wie folgt erhalten:
In einem zylindrischen Reaktionsgefäss (1,5 l Inhalt, Durchmesser 10 cm, Höhe 20 cm) mit dreifach tubuliertem Deckel, versehen mit Ankerrührer (Durchmesser 9 cm, Ankerhöhe 12 cm) und Thermometer werden 750 ml Wasser und 324 g $Na_2S \cdot 9H_2O$ auf 90–95 °C erhitzt, anschliessend innerhalb einer Stunde 81 g fein gesiebtes 1-Nitro-anthrachinon (Gehalt 73,9%) bei gleicher Temperatur eingerührt und abschliessend 30 Minuten bei 90–95 °C nachgerührt. Das Reaktionsgemisch wird abgesaugt, mit heissem Wasser neutral gewaschen und bei 60 °C im Vakuum getrocknet. Erhalten werden 71,3 g rohes 1-Aminoanthrachinon folgender Zusammensetzung:

| | |
|---|---|
| 72,5% | 1-Amino-anthrachinon = 98% d.Th. |
| 3,1% | 1,5-Diamino-anthrachinon |
| 2,8% | 1,8-Diamino-anthrachinon |
| 3,0% | 1,6-Diamino-anthrachinon |
| 3,1% | 1,7-Diamino-anthrachinon |
| 2,3% | Anthrachinon |
| 5,9% | 2-Amino-anthrachinon |
| 0,1% | 2,6- + 2,7-Diamino-anthrachinon. |

c) Das in b) eingesetzte 1-Nitro-anthrachinon wurde wie folgt erhalten:
In ein zylindrisches Reaktionsgefäss (1,5 l Inhalt, Durchmesser 10 cm, Höhe 20 cm) mit 4fach tubuliertem Deckel, versehen mit Ankerrührer (Durchmesser 9 cm, Ankerhöhe 12 cm) Thermometer, Kühler und Tropftrichter werden 150 g einer ausreagierten Nitriermischung, die 68 g rohes 1-Nitro-anthrachinon enthält, vorgelegt. In diese Vorlage trägt man bei 48–50 °C getrennt, aber unter synchroner Dosierung im Laufe von 3 Stunden bei einer Rührgeschwindigkeit von 200 Umdrehungen/Min. gleichmässig 500 g technisches Anthrachinon 99%ig) und 890 g Mischsäure folgender Zusammensetzung ein:

| | | |
|---|---|---|
| 351 | g | $H_2SO_4$ (100%ig) |
| 464 | g | $HNO_3$ (100%ig) |
| 76,5 | g | $H_2O$ |

Dabei wird eine Temperatur von 49–51 °C eingestellt. Nachdem 10–20% der Einsatzprodukte eingetragen sind, bildet sich ein Zweiphasensystem aus, das grosse Entmischungstendenz zeigt. Es besteht aus einer spezifisch schwereren, flüssigen, klaren, anorganischen Phase, bestehend aus Mischsäure, in der geringe Teile an Anthrachinon bzw. nitriertem Anthrachinon gelöst sind, und einer spezifisch leichteren, ebenfalls ganz oder überwiegend flüssigen, organischen Phase, bestehend aus Anthrachinon, Nitroanthrachinon, Salpetersäure und geringen Anteilen Schwefelsäure. Dieses Zweiphasensystem bleibt während der gesamten Zudosierzeit erhalten.

Nach erfolgter Zudosierung rührt man den Ansatz während einer Stunde bei 50 °C und abschliessend während 3 Stunden bei 60 °C nach. Dabei geht die flüssige organische Phase mit zunehmendem Nitrierungsgrad des Anthrachinons allmählich in eine feste Phase über, wobei eine fortlaufende Verdickung des Ansatzes zu beobachten ist, er bleibt aber bis zum Schluss der Reaktion gut rührbar.

Man rührt in 5 l kaltes Wasser ein, rührt ½ Stunde bei 60 °C nach, filtriert, wäscht mit heissem Wasser neutral und trocknet im Vakuum bei 120 °C. Die Ausbeute an so gewonnenem Produkt beträgt 685 g. Zusammensetzung:

1,9% Anthrachinon
73,9% 1-Nitro-anthrachinon
8,0% 2-Nitro-anthrachinon
3,2% 1,6-Dinitro-anthrachinon
3,4% 1,7-Dinitro-anthrachinon
3,6% 1,5-Dinitro-anthrachinon
3,4% 1,8-Dinitro-anthrachinon
0,2% 2,6- + 2,7-Dinitro-anthrachinon.

Die Ausbeute an 1-Nitro-anthrachinon beträgt 75% d.Th.

Beispiel 2

a) In einem 50 l Kessel werden 10 l Oleum (20%ig) vorgelegt und nacheinander 5 kg wasserfreies Natriumsulfat und 7,5 kg 1-Amino-anthrachinon (74,3%ig) eingetragen, dann wird unter Rühren innerhalb 1 Stunde auf 130 °C erhitzt und 2 Stunden bei gleicher Temperatur gehalten. Nun trägt man nacheinander 2,25 kg wasserfreies Natriumsulfat und 5,5 kg Oleum (20%ig) ein und rührt solange nach, bis in einer entnommenen Probe weniger als 3% unsulfoniertes Einsatzmaterial nachweisbar ist (ca. 3–4 Stunden erforderlich). Nun kühlt man auf 80 °C ab, setzt 10 g Jod zu und trägt bei dieser Temperatur im Laufe von 8 Stunden portionsweise insgesamt 3000 g Brom ein und hält bei dieser Temperatur solange, bis in einer entnommenen Probe <2% Aminsäure nachweisbar sind.

Nun kühlt man die Schmelze auf 50 °C ab und verdünnt bei 50–60 °C im Laufe einer Stunde mit 10 l Wasser, rührt eine weitere Stunde bei 50–60 °C nach, presst in eine Filterpresse ein und wäscht mit insgesamt 20 l $H_2SO_4$ (60%) portionsweise nach. Der Nutschkuchen wird in 100 l Wasser versetzt, auf 90 °C geheizt, 30 Minuten bei 90 °C gehalten, auf 30–40 °C abgekühlt, 30 Minuten gehalten, abgepresst und mit 100 l kalter 1,3%iger Natriumsulfatlösung gewaschen und getrocknet.

Erhalten werden 8,19 kg Bromaminsäure der Zusammensetzung:

85,1% 1-Amino-4-brom-anthrachinon-2-sulfonsäure
0,8% unsulfonierte 1-Amino-anthrachinone
1,1% 1-Amino-anthrachinon-2-sulfonsäure
1,0% andere Anthrachinonsulfonsäuren.

Die Ausbeute beträgt demnach 73% d.Th.

b) Das in a) eingesetzte 1-Amino-anthrachinon wurde wie folgt erhalten:

In eine Mischung aus 50 l Wasser und 27 kg NaSH-Lösung (18,5%ig) werden 54 kg 1-Nitro-anthrachinon (75,6%ig) innerhalb 1 Stunde bei 90 °C unter rühren eingetragen und eine weitere ½ Stunde bei 90–95 °C verrührt. Das Reaktionsgemisch wird heiss abgesaugt, mit 1%iger Natronlauge und abschliessend mit heissem Wasser neutral gewaschen und getrocknet.

Erhalten werden 46,3 kg 1-Amino-anthrachinon folgender Zusammensetzung:

74,3% 1-Amino-anthrachinon = 95,6% d.Th.
3,3% Anthrachinon
4,8% 2-Amino-anthrachinon
3,2% 1,5-Diamino-anthrachinon
2,9% 1,8-Diamino-anthrachinon
3,6% 1,6-Diamino-anthrachinon
3,6% 1,7-Diamino-anthrachinon
0,5% 2,6- + 2,7-Diamino-anthrachinon
———
96,2%

Statt 27 kg NaSH-Lösung (18,5%ig) kann man auch eine Mischung aus 9,8 kg $Na_2S \cdot 9H_2O$ und 12,8 kg NaSH-Lösung (18,5%ig) einsetzen.

c) Das in b) eingesetzte 1-Nitro-anthrachinon wurde wie folgt erhalten:

In das erste Element einer vierstufigen Kaskade (dreistufige Reaktionskaskade + Verdünnungskaskade) mit je 35 l Füllinhalt, versehen mit Eintragschnecke und Mischsäure-Dosierpumpe an Kaskade 1, Wasserdosierpumpe an Kaskade 4 und heiz- und kühlbaren Wassermänteln an Kaskaden 1–4, werden synchron bei 48–50 °C pro Stunde 10 000 g Anthrachinon (99%ig) und 16 100 g Mischsäure (Zusammensetzung: 52,1% $HNO_3$ (100%ig), 39,3% $H_2SO_4$ (100%ig), 8,6% $H_2O$) unter lebhaftem Rühren eindosiert. Gleichzeitig wird in Kaskade 4 die Wäsche 1 aus der Filtration des erhaltenen 1-Nitro-anthrachinon (ca. 18 900 g/h) synchron zudosiert.

Die Temperaturen in den einzelnen Elementen der Kaskade werden wie folgt eingestellt:

Kaskade 1  48–50 °C
Kaskade 2  54–56 °C

Kaskade 3  59–61 °C
Kaskade 4  58–62 °C

Die Aufarbeitung erfolgt so, dass der Überlauf von Kaskade 4 kontinuierlich auf einem Drehfilter filtriert und das Nutschgut in 2 unterschiedlichen Zonen mit Wasser von 60° gewaschen wird. In die erste Zone werden 17 900 g Wasser pro Stunde eingegeben und das dabei erhältliche Filtrat (Wäsche 1), das in einer Menge von ca. 18 900 g/h anfällt, zum Verdünnen in Kaskade 4 verwendet. In die zweite Zone werden 12 400 g Wasser pro Stunde eingespeist. Das dabei erhältliche Filtrat (Wäsche 2) kann der biologischen Abwasseraufbereitung zugeführt werden.

Das neutral gewaschene Nutschgut wird im Vakuum scharf getrocknet. Pro Stunde werden 12 550 g Produkt folgender Zusammensetzung erhalten:

    2,0%  Anthrachinon
    6,7%  2-Nitro-anthrachinon
  75,6%  1-Nitro-anthrachinon
    3,0%  1,6-Dinitro-anthrachinon
    3,8%  1,7-Dinitro-anthrachinon
    3,8%  1,5-Dinitro-anthrachinon
    3,4%  1,8-Dinitro-anthrachinon
    0,6%  2,7- + 2,6-Dinitro-anthrachinon
    1,1%  sonstige.

Die Ausbeute aus 1-Nitro-anthrachinone beträgt 77,9% d.Th.

Beispiel 3
a) In 120 ml Oleum (20%ig) werden unterhalb 80 °C 60 g Natriumsulfat eingerührt, dann trägt man 90 g 1-Amino-anthrachinon (87,5%ig) bei 50 bis 80 °C ein, erwärmt innerhalb 30 Minuten auf 130 °C und hält 2 Stunden bei dieser Temperatur. Nun setzt man 50 ml Oleum (30%ig) und 33 g Natriumsulfat zu, hält weitere 5 Stunden bei 130 °C. Chromatographisch sind nur noch weniger als 4% 1-Amino-anthrachinon nachweisbar.

Man kühlt ab auf 80 °C, setzt 0,2 g feinst verteiltes Jod und insgesamt 11,6 ml Brom in ca. 7 Stunden gleichmässig schnell bei 80 °C zu: Chromatoghraphisch sind jetzt nur noch weniger als 2% Aminsäure nachweisbar.

Nun treibt man überschüssiges Brom im Vakuum ab, gibt den Ansatz unter Rühren auf 4 l Wasser, setzt 640 g $Na_2SO_4$ zu, rührt 2 Std. bei Raumtemperatur nach, saugt ab, wäscht mit 200 ml 10%iger $Na_2SO_4$-Lösung und presst scharf ab. Erhalten werden 327 g Nutschkuchen. Der Nutschkuchen wird in 1200 ml Edelwasser aufgenommen, mit Natronlauge auf pH 9 gestellt und nach Zusatz von 8 g Aktivkohle und 8 g Kieselgur 20 Minuten lang auf 95 °C unter Rühren erwärmt. Es wird filtriert und mit 240 ml 95 °C heissem Edelwasser gewaschen. Man erhält 1610 ml Filtrat und nach Trocknung 18,4 g Rückstand. Das Filtrat wird bei 80 °C mit 120 g $Na_2SO_4$ ausgesalzen, 1 Stunde bei gleicher Temperatur nachgerührt, auf 30 °C abgekühlt, abgesaugt, mit 300 ml 1,5%iger $Na_2SO_4$-Lösung gewaschen und getrocknet.

Erhalten werden 126,6 g Na-Salz der Bromaminsäure mit einem Reingehalt von 84,4% (bezogen auf freie Säure).

b) Das in a) eingesetzte 1-Amino-anthrachinon wurde wie folgt erhalten:
145 g 1-Nitro-anthrachinon roh (75,1%ig), 145 g Nitrobenzol und 145 g Wasser werden im 1,3 l Rührautoklaven auf 190 °C erhitzt. Innerhalb einer ½ Stunde wird eine Lösung aus 19,7 g $NH_3$, 38 g $Na_2CO_3$, 15,7 g $NaHCO_3$ und 200 g Wasser zugepumpt. Man lässt noch 15 Minuten bei 190 °C reagieren und drückt die schwere organische Phase aus dem Autoklaven in ein Kristallisiergefäss. Nach dem Erkalten wird abgesaugt und getrocknet.

Ausbeute: 86 g 1-Amino-anthrachinon 87,5%ig ≙78,4% d.Th.

Verunreinigungen: 2,8% Anthrachinon, 1,8% 1-NA, 3,7% 1-Amino-5-nitro-anthrachinon, 1,1% 1,5-Diamino-anthrachinon, 1,0% 1,8-Diamino-anthrachinon.

c) Das in b) eingesetzte 1-Nitro-anthrachinon wurde wie folgt erhalten:
Man legt in einem zylindrischen Reaktionsgefäss 280 g $H_2SO_4$ (78%ig) vor, rührt darin 200 g Anthrachinon ein und tropft unter Rühren innerhalb 30 Minuten 235 g $HNO_3$ (98%ig) ein. Dabei steigt die Temperatur auf 47 °C an. Anschliessend tropft man gleichmässig schnell innerhalb einer Stunde eine Mischung von 137 g $H_2SO_4$ (96%ig) und 235 g $HNO_3$ (98%ig) zu und gibt während der Zugabe der letzten 75% der Mischsäure gleichmässig schnell 300 g Anthrachinon zu. Dabei wird eine Temperatur von 50–52 °C eingehalten. Man rührt eine Stunde bei 50 °C und 2 Stunden bei 60 °C nach. Nach der Aufarbeitung analog Beispiel 1c werden 619 g 1-Nitro-anthrachinon-Gemisch folgender Zusammensetzung erhalten:

    1,5%  Anthrachinon
    8,2%  2-Nitro-anthrachinon
  75,1%  1-Nitro-anthrachinon
    3,0%  1,6-Dinitro-anthrachinon
    2,9%  1,7-Dinitro-anthrachinon
    3,7%  1,5-Dinitro-anthrachinon
    2,9%  1,8-Dinitro-anthrachinon

Die Ausbeute an 1-Nitro-anthrachinon beträgt 76,6% d.Th.

Beispiel 4
a) Man führt die Reaktion, wie im Beispiel 3 beschrieben durch, setzt aber ein 86,8%iges 1-Amino-anthrachinon ein.

Die von überschüssigem Brom befreite Schmelze wird in 900 ml Eiswasser eingerührt, dabei steigt die Temperatur auf 30 °C an. Man saugt nach 30 Minuten bei dieser Temperatur ab und presst scharf ab. Den Nutschkuchen (262 g) nimmt man in 1200 ml Edelwasser auf, stellt mit NaOH auf pH 7 und anschliessend mit Soda auf pH 9, setzt 6 g A-Kohle und 10 g Kieselgur zu, erwärmt auf 95 °C, rührt bei dieser Temperatur 20 Minuten nach, filtriert und wäscht mit 240 ml $H_2O$ von 95 °C nach. Man erhält 1605 ml Filtrat und nach Trocknung 19,2 g Rückstand.

Man versetzt das Filtrat bei 80°C mit 160 g $Na_2SO_4$, rührt 1 Stunde bei 80°C nach, kühlt auf 30°C ab, filtriert, wäscht mit 300 ml 1,5%iger $Na_2SO_4$-Lösung und trocknet.

Erhalten werden 128,6 g Na-Salz der Bromaminensäure mit einem Reingehalt von 85,2% (berechnet auf freie Säure).

b) das in a) eingesetzte 1-Amino-anthrachinon wurde wie folgt erhalten:
85 g 1-Nitro-anthrachinon, aus Nitrobenzol umgelöst (88,2%ig), wird mit 145 ml Wasser im 0,7 l Rührautoklav auf 180°C geheizt. Innerhalb 5 Minuten wird eine Lösung aus 14,6 g $NH_4Cl$ in 240 ml 25%iger $NH_3$-Lösung zugepumpt. Man lässt noch eine Stunde reagieren, kühlt ab und saugt das Produkt ab. Nach dem Trocknen erhält man 75,4 g 1-Amino-anthrachinon 86,8%ig $\triangleq$ 97,4% d.Th.

c) Das in b) eingesetzte 1-Nitro-anthrachinon wurde wie folgt erhalten:
1) Reinigung des Nitroanthrachinon-Gemisches mit Nitrobenzol zur Gewinnung von Waschfiltrat
100 g 1-Nitro-anthrachinon folgender Zusammensetzung:

| | |
|---|---|
| 3,41% | Anthrachinon |
| 7,18% | 2-NA |
| 72,6 % | 1-NA |
| 4,09% | 1,6-DNA |
| 3,80% | 1,7-DNA |
| 3,19% | 1,5-DNA |
| 3,07% | 1,8-DNA |
| 0,12% | $H_2SO_4$, |

erhalten nach dem Verfahren der DT-OS 22 33 185,
werden zusammen mit 108 g Nitrobenzol auf 150° erwärmt, bis eine klare Lösung entsteht, anschliessend wird die Lösung innerhalb 30 Minuten auf 65°C abgekühlt und dann 3 Stunden bei 65°C gerührt. Das Kristallisat wird nun bei 65°C abgesaugt, nach Wechsel der Vorlage mit 108 g kaltem Nitrobenzol gewaschen (= Waschfiltrat) und der Rückstand bei 100°C getrocknet.
2) Reinigung des Nitroanthrachinon-Gemisches unter Einsatz von Waschfiltrat als Lösungsmittel
100 g 1-Nitro-anthrachinon obiger Zusammensetzung wird mit dem Waschfiltrat aus 1) anstelle von 108 g Nitrobenzol nach der unter 1) angegebenen Vorschrift behandelt. Man erhält trockenes, vorgereinigtes 1-Nitro-anthrachinon (= Kristallisat) und Mutterlauge sowie Waschfiltrat. Die nach dieser Vorschrift erhaltenen Waschfiltrate werden jeweils als Lösungsmittel für den nächsten Kristallisationsansatz eingesetzt.

Nach zweimaligem Wiedereinsatz von Waschfiltrat (= 3. Kristallisation) haben sich die Kristallisationsgleichgewichte eingestellt. Bei den folgenden 6 Kristallisationen wurde folgendes mittlere Versuchsergebnis erhalten (pro 100 g Einsatz obiger Zusammensetzung):
Kristallisat: 76,6 g 8,2%iges 1-Nitro-anthrachinon, entsprechend einer Trennausbeute von 93,1%.

Analyse:
| | |
|---|---|
| 0,45±0,08% | Anthrachinon |
| 0,40±0,09% | Nitroanthrachinon |
| 88,20±1,05% | 1-Nitroanthrachinon |
| 0,74±0,08% | 1,6-Dinitroanthrachinon |
| 3,16±0,19% | 1,7-Dinitroanthrachinon |
| 3,39±0,15% | 1,5-Dinitroanthrachinon |
| 1,16±0,19% | 1,8-Dinitroanthrachinon |
| 0,02±0,01% | $H_2SO_4$ |

Beispiel 5
a) Man arbeitet wie im Beispiel 2 beschrieben, setzt aber ein 86,7%iges 1-Amino-anthrachinon ein.

Der Nutschkuchen aus der Filterpresse wird wie folgt aufgearbeitet:
Es wird in 100 l Wasser eingerührt, bei 30–40°C filtriert und mit kaltem Wasser bis auf pH 2,5–3 im Ablauf gewaschen.

Nun löst man das Nutschgut bei 90–95°C in der erforderlichen Menge Wasser (ca. 130 l erforderlich), setzt je 250 g A-Kohle und Kieselgel zu, filtriert, wäscht den Rückstand mit heissem Wasser nach und salzt das Filtrat bei 30–40°C mit 3–4 kg $Na_2SO_4$ aus, erwärmt die Fällung nochmals bis 80°C, rührt bis 40° kalt und filtriert.

Erhalten werden 8,9 kg Bromaminsäure in Form des Na-Salzes der Zusammensetzung:

| | |
|---|---|
| 87,7% | 1-Amino-4-brom-anthrachinon-2-sulfonsäure |
| 0,4% | unsulfierte 1-Amino-anthrachinone |
| 0,9% | 1-Amino-anthrachinon-2-sulfonsäure |
| 1,1% | andere Anthrachinonsulfonsäuren. |

b) Das in a) eingesetzte 1-Amino-anthrachinon wurde wie folgt erhalten:
100 g 1-Nitro-anthrachinon der in Beispiel 4 c1 genannten Zusammensetzung werden in 500 ml Wasser während 12 Stunden kalt verrührt, anschliessend setzt man bei 90°C im Laufe von mehreren Stunden eine Lösung von 75 g $Na_2SO_3$ in 200 ml Wasser zu, hält insgesamt 8 Stunden bei dieser Temperatur und gibt anschliessend 350 g $Na_2S \cdot 9H_2O$ zu und hält bei dieser Temperatur solange, bis chromatographisch keine Nitroverbindungen mehr nachweisbar sind (ca. 2 Stunden erforderlich). Das Reaktionsgemisch wird heiss abgesaugt, mit heissem Wasser neutral gewaschen und getrocknet.

Erhalten werden 72 g rohes 1-Amino-anthrachinon der Zusammensetzung:

| | |
|---|---|
| 86,7% | 1-Amino-anthrachinon = 86,2% d.Th. |
| 1,9% | 1,5-Diamino-anthrachinon |
| 1,2% | 1,8-Diamino-anthrachinon |
| 0,9% | 1,6-Diamino-anthrachinon |
| 1,2% | 1,7-Diamino-anthrachinon |
| 1,4% | 2-Amino-anthrachinon |
| 2,9% | Anthrachinon. |

Beispiel 6
a) In einem 50 l Kessel mit Ankerrührer werden 10 l 20%iges Oleum vorgelegt. Dann trägt man nacheinander 5 kg wasserfreies Natriumsulfat

15 0 001 087 16

(Temperaturanstieg auf 60–70°C) und 7,5 kg 1-Aminoanthrachinon (73,4%ig) ein (Temperaturanstieg auf 75°C). Nun heizt man innerhalb 1 Stunde auf 130°C und rührt noch 2 Stunden bei dieser Temperatur. Anschliessend setzt man noch 2,25 kg $Na_2SO_4$ und 5,5 l 20%iges Oleum zu und führt die Reaktion durch Nachrühren bei 130°C zu Ende. Der Endpunkt ist erreicht, wenn in einer Probe der Schmelze dünnschichtchromatographisch nur noch weniger als 5% Aminoanthrachinon nachweisbar sind (3 bis 4 Stunden). Dann wird auf 80°C gekühlt und nach Zusatz von 10 g Jod mit 3,1 kg Brom portionsweise bromiert. Das überschüssige Brom wird mit Stickstoff ausgeblasen. In einem weiten 100 l fassenden Kessel werden 20 l einer kalten, 30%igen Schwefelsäure vorgelegt. Sobald die Temperatur der Reaktionsmasse auf 50–60°C abgekühlt ist, wird der Inhalt in den weiten Kessel ausgetragen, wobei die Temperatur auf 70–80°C steigt. Man lässt noch 1–2 Stunden rühren und presst bei 50–60°C ab. Der Presskuchen wird mit 15 l 60%iger Schwefelsäure gewaschen, in 20 l Wasser verrührt und erneut über eine Presse filtriert. Der Presskuchen wird in 100 l Wasser bei 80–85°C gelöst und die Lösung mit 0,6 kg Aktivkohle und 0,6 kg Kieselgel geklärt. Nach der Klärung wird die Bromaminsäure mit Natronlauge (4,7 l 50%ig) bei einem pH von 8–9 bei 80°C ausgefällt. Die Fällung wird durch Zusatz von 2 kg Natriumsulfat vervollständigt und die Suspension noch 4 Stunden bei 80°C gerührt. Nach dem Erkalten auf 20°C lässt man erneut einige Zeit rühren und isoliert das Natriumsalz der Bromaminsäure über eine Filterpresse. Gewaschen wird mit 100 l 1,5%iger Natriumsulfatlösung. Nach der Trocknung werden 7,5 kg Bromaminsäure 84,8%ig ≙67,4% der Theorie erhalten.

b) Das in a) eingesetzte 1-Aminoanthrachinon wird wie in Beispiel 2b) erhalten.

**Patentansprüche:**

1) Verfahren zur Herstellung von 1-Amino-4-bromanthrachinon-2-sulfonsäure oder deren Alkalisalze, dadurch gekennzeichnet, dass man
a) verunreinigtes 1-Nitroanthrachinon mit einem Reingehalt von 70 bis 90% an 1-Nitroanthrachinon durch Reduktion mit Natriumsulfid und/oder Natriumhydrogensulfid oder durch Austausch der Nitrogruppe mit Ammoniak unter Druck in 1-Amino-anthrachinon überführt,
anschliessend
b) das rohe 1-Amino-anthrachinon bei Temperaturen im Bereich von 100 bis 150°C zuerst mit Oleum unter Zusatz von Alkalimetallsulfaten und dann bei Temperaturen im Bereich von 60 bis 100°C mit Brom behandelt
und danach
c) die 1-Amino-4-bromanthrachinon-2-sulfonsäure oder deren Alkalisalze aus dem Reaktionsgemisch durch Einstellen der Schwefelsäure-Konzentration auf einen Schwefelsäure-Gehalt von 60 bis 85 Gew.-% oder durch Einrühren des Reaktionsgemisches in gegebenenfalls Alkalisulfat-haltiges Wasser ausfällt, abfiltriert und gegebenenfalls die 1-Amino-4-bromanthrachinon-2-sulfonsäure oder deren Alkalisalze aus Wasser umlöst.

2) Verfahren nach Anspruch 1, dadurch gekennzeichnet, das man das rohe 1-Amino-anthrachinon mit 5 bis 40%igem Oleum versetzt.

3) Verfahren nach Ansprüche 1 und 2, dadurch gekennzeichnet, dass man die 2- bis 5fache Gewichtsmenge Oleum, bezogen auf Amino-anthrachinone, dem Reaktionsgemisch zusetzt.

4) Verfahren nach Ansprüche 1 bis 3, dadurch gekennzeichnet, dass man als Alkalimetallsulfate Natrium-und/oder Kaliumsulfat verwendet.

5) Verfahren nach Ansprüche 1 bis 4, dadurch gekennzeichnet, dass man 0,4 bis 1,4 Gewichtsteile Alkalimetallsulfat pro Teil Amino-anthrachinone dem Reaktionsgemisch zugibt.

6) Verfahren nach Ansprüche 1 bis 5, dadurch gekennzeichnet, dass man das Reaktionsgemisch mit mindestens 0,5 Moläquivalente Brom, bezogen auf 1 Mol 1-Aminoanthrachinon, behandelt.

7) Verfahren nach Ansprüche 1 bis 5, dadurch gekennzeichnet, dass man das Reaktionsgemisch mit 0,6 bis 0,9 Moläquivalente Brom, bezogen auf 1 Mol 1-Aminoanthrachinon, behandelt.

**Patent Claims**

1. Process for the preparation of 1-amino-4-bromo-anthraquinone-2-sulphonic acid, or alkali metal salts thereof, characterised in that a) impure 1-nitro-anthraquinone, which has a pure content of 1-nitro-anthraquinone of from 70% to 90%, is converted into 1-amino-anthraquinone by reduction with sodium sulphide and/or sodium bisulphide or by replacing the nitro group using ammonia under pressure, then b) the crude 1-amino-anthraquinone is first treated with oleum at temperatures in the range from 100 to 150°C, alkali metal sulphates being added, and the product is then treated with bromine at temperatures in the range from 60 to 100°C, and thereafter c) the 1-amino-4-bromanthraquinone-2-sulphonic acid, or alkali metal salts thereof, is precipitated from the reaction mixture by adjusting the sulphuric acid concentration to a sulphuric acid content of 60% to 85% by weight or by stirring the reaction mixture into water, which optionally contains alkali metal sulphate, and is filtered off and the 1-amino-4-bromoanthraquinone-2-sulphonic acid, or alkali metals salts thereof, is appropriately redissolved in water and reprecipitated.

2. Process according to Claims 1, characterised in that 5% to 40% strength oleum is added to crude 1-amino-anthraquinone.

3. Process according to Claims 1 and 2, characterised in that 2 times to 5 times the amount by weight of oleum, relative to amino-anthraquinones, are added to the reaction mixture.

4. Process according to Claims 1 to 3, characterised in that sodium sulphate and/or potassium sulphate are used as the alkali metal sulphates.

5. Process according to Claims 1 to 4, charac-

9

terised in that 0.4 to 1.4 parts by weight of alkali metal sulphate per part of amino-anthraquinones are added to the reaction mixture.

6. Process according to Claims 1 to 5, characterised in that the reaction mixture is treated with at least 0.5 molar equivalent of bromine, relative to 1 mol of 1-amino-anthraquinone.

7. Process according to Claims 1 to 5, characterised in that the reaction mixture is treated with 0.6 to 0.9 molar equivalent of bromine, relative to 1 mol of 1-amino-anthraquinone.

## Revendications

1. Procédé de préparation de l'acide 1-amino-4-bromo-anthraquinone-2-sulfonique ou de ses sels alcalins, caractérisé en ce que
a) on convertit de la 1-nitro-anthraquinone impure ayant un degré de pureté de 70 à 90% en 1-nitro-anthraquinone, par réduction avec du sulfure de sodium et/ou de l'hydrogénosulfure de sodium ou par échange du groupe nitro avec de l'ammoniaque sous pression, en 1-amino-anthraquinone, ensuite
b) on traite de la 1-amino-anthraquinone brute à des températures dans l'intervalle de 100 à 150°C, d'abord avec de l'oléum avec addition de sulfates de métaux alcalins, puis à des températures dans l'intervalle de 60 à 100°C avec du brome, et par la suite
c) on précipite l'acide 1-amino-4-bromo-anthraquinone-2-sulfonique ou ses sels alcalins à partir du mélange de réaction par réglage de la concentration en acide sulfurique à une teneur en acide sulfurique de 60 à 85% en poids ou par incorporation sous agitation du mélange de réaction dans de l'eau qui contient éventuellement un sulfate alcalin, on sépare par filtration et l'on recristallise éventuellement l'acide 1-amino-4-bromo-anthraquinone-2-sulfonique ou ses sels alcalins à partir d'eau.

2. Procédé selon la revendication 1, caractérisé en ce qu'on ajoute à la 1-amino-anthraquinone brute de l'oléum à 5-40%.

3. Procédé selon l'une quelconque des revendications 1 et 2, caractérisé en ce qu'on ajoute la quantité double à quintuple en poids d'oleum, par rapport aux amino-anthraquinones, au mélange de réaction.

4. Procédé selon l'une quelconque des revendications 1 à 3, caractérisé en ce qu'on utilise en tant que sulfates de métaux alcalins du sulfate de sodium et/ou sulfate de potassium.

5. Procédé selon l'une quelconque des revendications 1 à 4, caractérisé en ce qu'on ajoute 0,4 à 1,4 partie en poids de sulfate de métal alcalin par partie d'amino-anthraquinone au mélange de réaction.

6. Procédé selon l'une quelconque des revendications 1 à 5, caractérisé en ce qu'on traite le mélange de réaction avec au moins 0,5 équivalent molaire de brome par mole de 1-amino-anthraquinone.

7. Procédé selon l'une quelconque des revendications 1 à 5, caractérisé en ce qu'on traite le mélange de réaction avec 0,6 à 0,9 équivalent molaire de brome par mole de 1-amino-anthraquinone.